# EUROPEAN PATENT SPECIFICATION

(11) **EP 1 970 433 B1**
(45) Date of publication and mention of the grant of the patent: **02.09.2015**
(21) Application number: 08152764.0
(22) Date of filing: 14.03.2008
(51) Int. Cl.: C12M 1/00

(54) **An algae microbe photosynthesis reaction system and method thereof**
Algen-Mikroben-Photosynthese-Reaktionssystem und Verfahren dafür
Système de réaction de photosynthèse dans des algues microscopiques et système et procédé correspondants

(30) Priority: 16.03.2007 DE 102007012745
(43) Date of publication of application: 17.09.2008
(73) Proprietor: Lu, Chao-Hui, Hsin Chu City, Taiwan (CN)
(72) Inventor: Lu, Chao-Hui, Hsin Chu City, Taiwan (CN)
(74) Representative: Viering, Jentschura & Partner Patent- und Rechtsanwälte

(56) References cited:
- EP-A- 0 239 272
- WO-A-96/23865
- US-B1- 7 056 725

## Description

### BACKGROUND OF THE INVENTION

### 1. Field of the Invention

The invention generally relates to a photosynthesis reaction system and method and particularly to an algae microbe photosynthesis reaction system and method thereof.

### 2. Description of the Related Art

Blue-Green Algae (known as Spirulina and Arthrospira) or Haematococcus Pluvialis Flotow which contains rich nutritious ingredients including proteins, minerals, vitamins, enzymes, antioxidants, astaxanthin, and etc; so therefore have recently been widely recommended as health foods. Spirulina algae's nutrient solution is used by photosynthesis reaction system which supplies the required nutrition for algae cells to grow and ventilates oxygen generated within the nutrient solution so that Spirulina algae can be cultivated in mass.

A conventional photosynthesis reaction system for Spirulina is performed in an air-exposed culture pool. The nutrient solution of Spirulina is placed into the exposed pool for photosynthesis. However, the pool requires a large area and consumes a lot of energy. In addition, the weather can be a restricting factor. The obtained algae are also easily polluted, deteriorating its quality.

Another conventional photosynthesis reaction system, such as disclosed in CN Patent NO 95219504.6, is a photosynthetic reaction system for spirulina, comprising a photosynthetic reaction unit and a vertical dual-spiral pipe. The photosynthetic reaction unit and the vertical dual-spiral pipe are transparent for the photosynthesis of the nutrient solution therein. The photosynthetic reaction unit has a bubbling plate and a thermal exchanger to ventilate oxygen generated in the nutrient solution and controls the temperature of the nutrient solution. The reaction system aims at providing a closed circulation environment to overcome shortages of large culture pool. However, this kind of reaction system still has problem of difficulty in oxygen ventilation, temperature control over the nutrient solution at a required level and maintenance of the photosynthetic reaction unit, which would decrease the effect of photosynthesis and Spirulina quality, therefore it is limited in terms of suitability for mass industrial cultivation.

WO 96/23865 describes another process for cultivating microalgae in a closed circuit.

Those prior systems have difficulties in practical use and therefore are limited in terms of mass production. Therefore, there is a need of a new photosynthesis reaction method and system which overcome the prior problems.

### SUMMARY OF THE INVENTION

An object of the invention is to provide a method and a system for photosynthesis reaction of algae microbe with reduced use in area, reduced energy requirement, no restrain from everyday weather, and prevention from pollution in order to maintain the quality of algae obtained, and further more ensures the ventilation of oxygen generated by the algae and the accurate control of the temperature and light intensity of the nutrient solution, so that algae can be cultivated quickly in mass with lower cost.

In order to achieve the objectives supra, the method of the invention comprises (a) providing a photosynthesis reaction unit, comprising a transparent piping, a communicating unit (communicating in the present invention strictly means connecting, and should not be confused with telecommunication), a collecting valve, a pressured liquid transport unit with a transport pipe, an oxygen jet device with a liquid inlet, and a connecting pipe; and (b) injecting an algae and microbe nutrient solution into the transparent piping, circulating the nutrient solution within the transparent piping for photosynthesis to generate oxygen, wherein the nutrient solution flows to the communicating unit, the collecting valve, and the pressured liquid transport unit; (c) determining an included angle on a horizontal cross-sectional surface of the oxygen jet device between an axial line of the transport pipe and a normal line passing through a central point of the oxygen jet device, wherein the included angle is adjustable according to different kinds of algae; (d) opening the pressured liquid transport unit to force the nutrient solution flow toward the oxygen jet device from the transport pipe, wherein the nutrient solution flows into the oxygen jet device from the transport pipe via the liquid inlet along the included angle and caused a shear force on the algae, and is splashed into the oxygen jet device to form a spinning splash by which the oxygen is ventilated, wherein the shear force is reduced when the included angle is smaller; (e) collecting the nutrient solution in the oxygen jet device and the connecting pipe; (f) flow the nutrient solution into the transparent piping for further photosynthesis; and (g) taking the nutrient solution from the collecting valve.

Another aspect of the invention is to provide a system for photosynthesis reaction of algae microbe which comprises a photosynthesis reaction unit, which is a transparent piping; a communicating unit, connected to an exit of the transparent piping; a collecting valve, connected to an exit of the communicating unit; a pressured liquid transport unit, an entrance of which is connected to the collecting valve, and the pressured liquid transport unit has a transport pipe; an oxygen jet device, being a hollow pipe and comprising the oxygen ventilating column and a liquid collector at a lower end thereof, the oxygen ventilating column having a liquid inlet, an upper ventilator, and a pipe wall, the exit of the transport pipe communicating with the liquid inlet, wherein the exit of the transport pipe being connected to the liquid inlet along an included angle defined on a horizontal cross-sectional surface of the oxygen jet device between an axial line of the transport pipe and a normal line passing through a central point of the oxygen jet device, wherein the included angle is adjustable according to different kinds of algae, the upper ventilator being located at a top of the oxygen ventilating column, and the pipe wall extending downward from the upper ventilator; and a connecting pipe, being a close pipe and connected the liquid collector to the photosynthesis reaction unit.

The enclosed branched configuration formed by the photosynthesis reaction unit, the communicating unit, the collecting valve, the pressured liquid transport unit, the transport pipe, the oxygen jet device and the connecting pipe allow the enclosed nutrient solution to circulate vertically therein for photosynthesis and oxygen ventilation. Its unique features such as small use of area, reduced energy requirement and no restrain from everyday weather greatly increases the algae yield and quality because the mostly enclosed algae can be protected from pollutions. Furthermore, light supplement unit can be added to one or both sides of the transparent piping for proper temperature and light intensity adjustment.

The arrangement of the liquid inlet, the upper ventilator and the pipe wall makes the oxygen the oxygen formed in the nutrient solution easy to ventilate, thereby increasing the yield and is favorable for mass production.

Furthermore, a temperature controlling unit is further mounted between the transparent piping and the pressured liquid transport unit to control the temperature within in the proper range for algae cultivation. A switch valve is mounted between the connecting piping and the liquid collector to drain out the nutrient solution when cleaning or to collect a nutrient solution sample.

To provide a further understanding of the invention, the following detailed description illustrates embodiments and examples of the invention; this detailed description being provided is for illustrative purpose only and is not meant to limit the scope of the invention.

### BRIEF DESCRIPTION OF THE DRAW INGS

FIG. 1 is a flow chart of a method for photosynthesis reaction of algae microbe according to one embodiment of the invention;
FIG. 2 is a schematic view of a system for photosynthesis reaction of algae microbe according to a first embodiment of the invention;
FIG. 3 is a schematic view of a system for photosynthesis reaction of algae microbe according to a second embodiment of the invention;
FIG. 4 is a top view (1) of a transport pipe and an oxygen ventilating column of a system for photosynthesis reaction of algae microbe according to one embodiment of the invention;
FIG. 4A is a top view (2) of a transport pipe and an oxygen ventilating column of a system for photosynthesis reaction of algae microbe according to one embodiment of the invention;
FIG. 4B is a top view (3) of a transport pipe and an oxygen ventilating column of a system for photosynthesis reaction of algae microbe according to one embodiment of the invention;
FIG. 4C is a top view (4) of a transport pipe and an oxygen ventilating column of a system for photosynthesis reaction of algae microbe according to one embodiment of the invention;
FIG. 5 is another top view of a transport pipe and an oxygen ventilating column of a system for photosynthesis reaction of algae microbe according to one embodiment of the invention;
FIG. 6 is a schematic view of a system for photosynthesis reaction of algae microbe according to a third embodiment of the invention;
FIG. 7 is a schematic view of a system for photosynthesis reaction of algae microbe according to a fourth embodiment of the invention;
FIG. 8 is a schematic view of a system for photosynthesis reaction of algae microbe according to a fifth embodiment of the invention;
FIG. 9 is a schematic view of a system for photosynthesis reaction of algae microbe according to sixth embodiment of the invention;
FIG. 10 is a schematic view of a system for photosynthesis reaction of algae microbe according to a seventh embodiment of the invention; and
FIG. 11 is a schematic view of a system for photosynthesis reaction of algae microbe according to one embodiment of the invention.

### DETAILED DESCRIPTION OF THE EMBODIIMENTS

Wherever possible in the following description, like reference numerals will refer to like elements and parts unless otherwise specified.

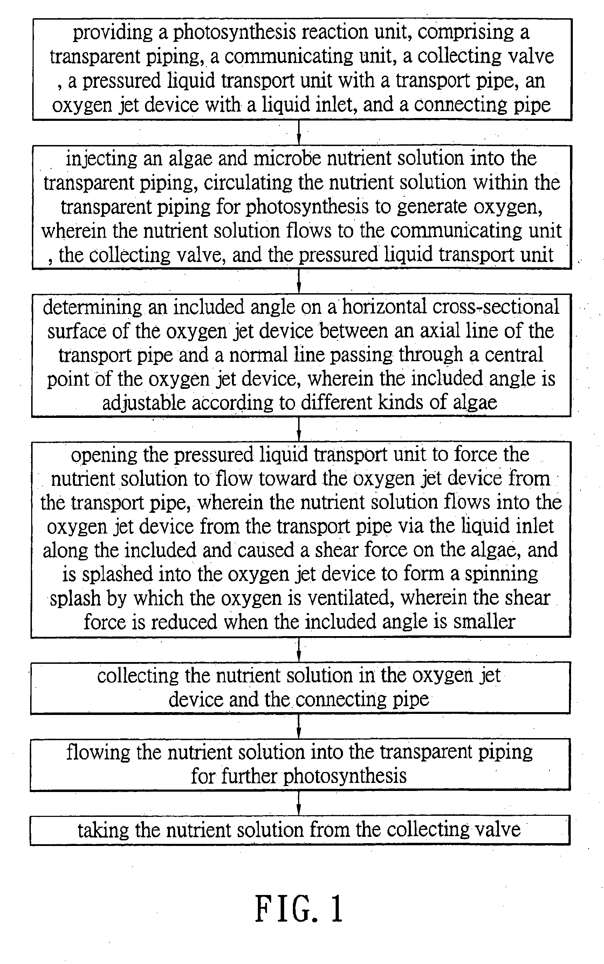

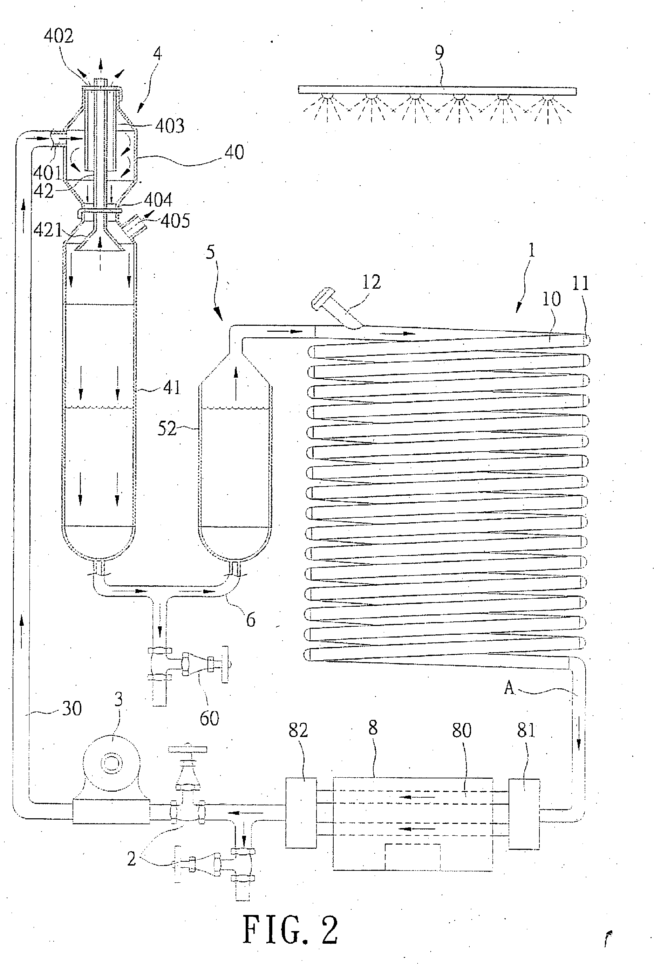

Referring to FIG. 1 and FIG. 2, an algae microbe photosynthesis reaction system and method thereof according to one embodiment of the invention are used to cultivate algae such as Spirulina or Haematococcus Pluvialis Flotow. The method comprises the following steps.

Step (a) provides a photosynthesis reaction unit 1 which comprises a transparent piping made of transparent material such as glass, a communicating unit A, a collecting valve 2, a pressured liquid transport unit 3 having a transport pipe 30, an oxygen jet device 4 having a liquid inlet 401, and a connecting pipe 5. The transparent piping (i.e. photosynthesis reaction unit 1), the communicating unit A, the collecting valve 2, the pressured liquid transport unit 3, the oxygen jet device 4 and the connecting pipe 5 are connected to one another to form the algae microbe photosynthesis system according to the invention.

At Step (b), nutrient solution and algae seed are injected into the transparent piping. The nutrient solution flows through the transparent piping for photosynthesis to generate oxygen. The nutrient solution flows toward the communicating unit A and then flows to the collecting valve 2 and the pressured liquid transport unit 3.

At step (c), an included angle on a horizontal cross-sectional surface of the oxygen jet device 4 between an axial line of the transport pipe 30 and a normal line N passing through a central point of the oxygen jet device 4 is determined, wherein the included angle is adjustable according to different kinds of algae.

The transparent piping is a spiral transparent piping. Therefore, the nutrient solution flows up and down to circulate within the transparent piping so that the algae microbe absorbs sufficient light for photosynthesis for quick growth.

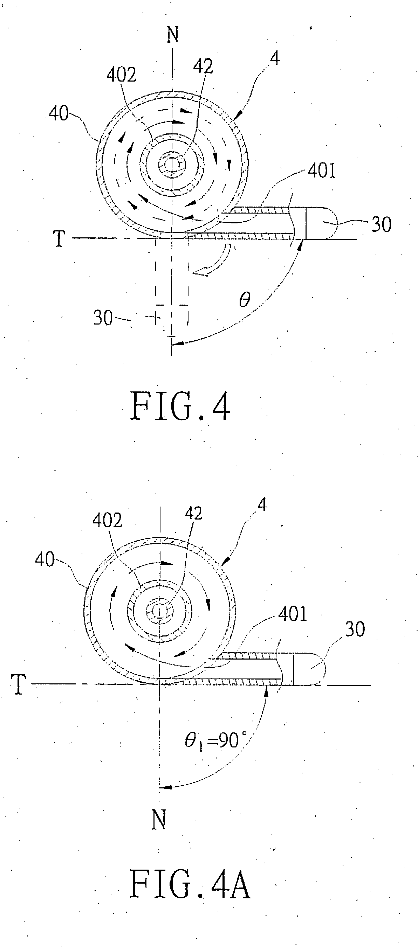

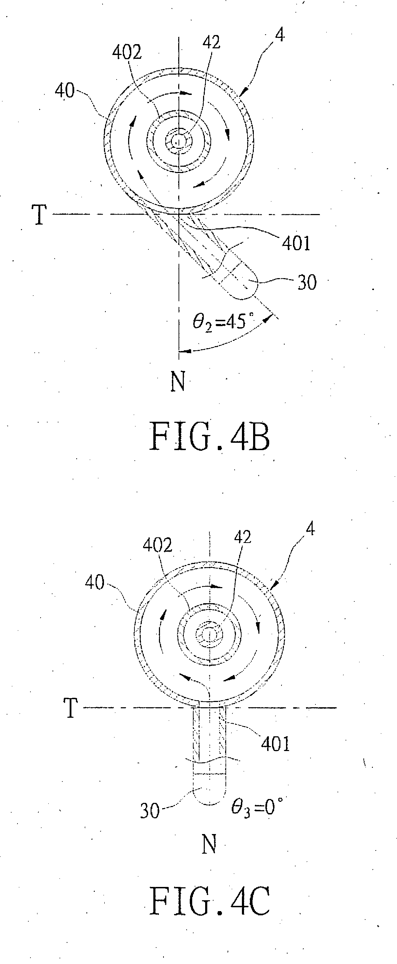

At Step (d), the pressured liquid transport unit 3 is opened to force the nutrient solution to flow toward the oxygen jet device 4 from the transport pipe 30. The nutrient solution flows into the oxygen jet device 4 from the transport pipe 30 via the liquid inlet 401 along the included angle and caused a shear force on the algae, as shown in FIG. 4. The nutrient solution is splashed into the oxygen jet device 4 to form a spinning splash by which the oxygen is ventilated, wherein the shear force is reduced when the included angle is smaller. Specifically, the nutrient solution is splashed into an oxygen ventilating column 40 to form the spinning splash which is then ventilated through an upper ventilator 402. The nutrient solution drops to be collected by a neck 404 and then go on to hit an extension 421 of an exhaust 42 to form another splash so that oxygen is exhausted from a side ventilator 405. Finally, the nutrient solution drops to be collected inside a liquid collector 41 so that oxygen can be ventilated out from a top of the exhaust 42. Thereby, most of oxygen in the nutrient solution is ventilated out from the nutrient solution for further photosynthesis of algae microbe.

At Step (e), the nutrient solution is collected in the oxygen jet device 4 and the connecting pipe 5.

At Step (f), the nutrient solution flows into the transparent piping for further photosynthesis.

The nutrient solution is collected in the liquid collector 41 and flows into the connecting piping 5 via a connector 6 for further photosynthesis reaction. The higher liquid level of the nutrient solution collected by the liquid collecting column 41, as compared to an upper layer in the transparent piping of the photosynthesis reaction unit 1, creates a potential drop to generate the pressure so that the algae nutrient solution automatically flows into the transparent piping via the connecting pipe 5.

At Step (g), after the nutrient solution flows through the transparent piping and the communicating unit A, the nutrient solution is taken from the collecting valve 2.

Furthermore, a temperature controlling unit 8 is provided in a manner such that the nutrient solution flows toward the collecting valve 2 and the pressured liquid transport unit 3 after flowing through the communicating unit A and the temperature controlling unit 8. Thereby, the temperature of the nutrient solution can be controlled.

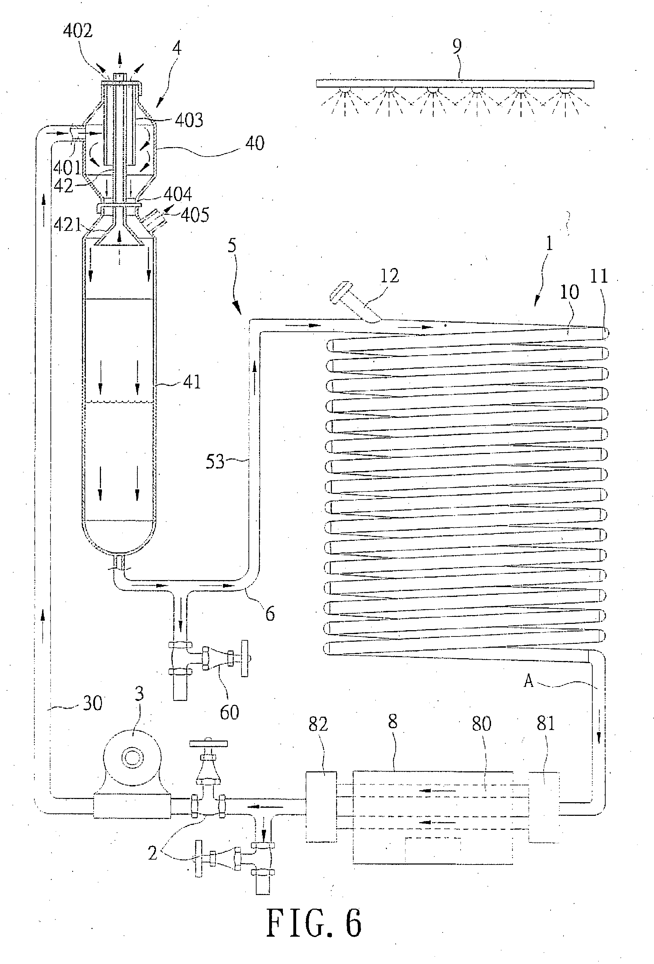

Furthermore, a water sprayer 9 is used for Step (b) and Step (g). The water sprayer 9 sprays water over the transparent piping as needed to reduce the temperature of the nutrient solution inside the transparent piping. A light supplement unit B is further provided for Step (b) and Step (g) as shown in FIG. 6. The light supplement unit B can be properly adjusted for the light intensity or the light source needed for the nutrient solution in the transparent piping. The photo synthesis unit 1 comprises a plurality of straight pipes 10 and a plurality of bent pipes 11. The straight pipes 10 and the bent pipes 11 are arranged in series in turns to form a dual-row tilt spiral transparent piping. An auxiliary opening 12 is formed at an upper most part of the transparent piping.

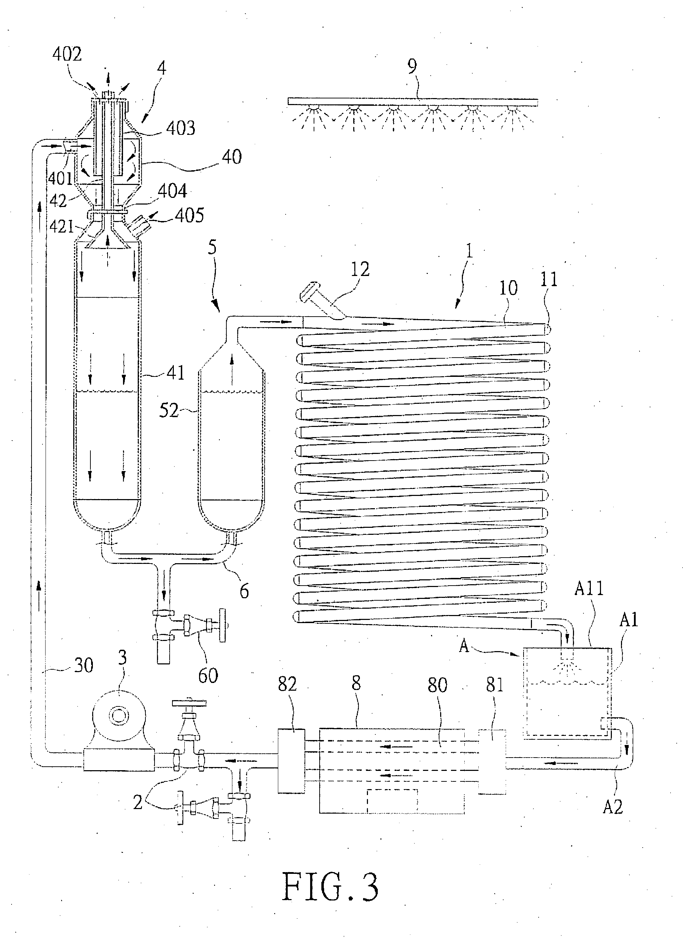

The communicating unit A is connected to an exit of the transparent piping. The communicating unit A can be a connecting pipe which connects an exit of the transparent piping to an entrance of the pressured liquid transport unit 3 in order to allow the nutrient solution to directly flows into the pressured liquid transport unit 3. Alternatively, as shown in FIG. 3, the communicating unit A comprises a liquid collecting sink A1 having an upward opening A11 and a liquid flowing pipe A2. The shape of the liquid collecting sink A1 is not specifically limited, and the liquid collecting sink A1 is made of the transparent material or opaque material. The liquid collecting sink A1 corresponds to an exit of the transparent piping for collecting the nutrient solution. The liquid flowing pipe A2 communicates with the liquid collecting sink A1 and an entrance of the collecting valve 2 so that the collected nutrient solution flows towards the pressure liquid transport unit 3. An opening A11 of the liquid collecting sink A1 can be covered by a transparent element to prevent any dusts from being dropping into the nutrient solution. Alternatively, the liquid collecting sink A1 is placed into a conservatory for preventing any dusts.

The collecting valve 2 communicates with the exit of the communicating unit A for collecting the nutrient solution flowing within the transparent piping. The pressured liquid transport unit 3 is a pressured liquid pump whose entrance communicates with the collecting valve 2. The pressured liquid transport unit 2 has a transport pipe 30.

The oxygen jet device 4 is a hollow pipe comprising the oxygen ventilating column 40 and a liquid collector 41. The oxygen ventilating column 40 is made of stainless steel. The liquid collector 41 is made of transparent glass or acrylic, or an opaque material such as stainless steel. At an upper section of the oxygen ventilating column 40 formed the liquid inlet 401, the upper ventilator 402, and a pipe wall 403. The transport pipe 30 communicates with the liquid inlet 40. The upper ventilator 402 is located at the top of the oxygen ventilating column 40. The pipe wall 403 extends downward from the upper ventilator 402 opposite to an interior of the liquid inlet n401.

Furthermore, referring to FIG. 4, in order to define different culture conditions for different algae, the oxygen jet device 4 has a normal line N and a tangent line T intersecting the normal line N. The exit of the transport pipe 30 communicates with the liquid inlet 401 along an angleθbetween the tangent line T and the normal line N. The angleθcan be 0-90 degrees. Preferably, the angleθ of the transport pipe 30 is in the range of 90-10 relative to the tangent line T.

The oxygen ventilating column 40 has a neck 404 and a side ventilator 405 at its middle section. The side ventilator 405 is located under the neck 404. The oxygen ventilating device 4 further comprises the exhaust 42 assembled inside the oxygen ventilating column 40. An upper end of the exhaust 42 penetrates through the pipe wall 403. A lower end of the exhaust 42 has the extension 421 opposite to the interior of the side ventilator 405. The extension 421 can be formed, with any shape, depending on requirements of algae species.

The connecting pipe 5 is a close pipe which can be an extending pipe 52. An end of the connecting pipe 5 connects to the photosynthesis reaction unit 1. The entrance of the transparent piping is bent downward to connect to the other end of the connecting pipe5.

The algae photosynthesis system further comprises a connector 6 connecting a bottom of the liquid collector 41 to a bottom of the connecting pipe 5. The connector 6 further has a switch valve 60.

When the algae microbe photosynthesis system according to the invention is used, the algae microbe algae seeds and algae microbe nutrient solution are poured into the auxiliary opening 12 of the transparent piping. The nutrient solution and the algae microbe flow through the transparent piping to conduct the photosynthesis which generates oxygen. The nutrient solution flows to the communicating unit A, and then flows to the collecting valve 2 and the pressured liquid transport unit 3. The pressured liquid transport unit 3 forces the nutrient solution to flow to the oxygen jet device 4 from the communicating unit A.

When the liquid inlet 401 is located at the upper section of the oxygen ventilating column 40, the nutrient solution is jetted into the oxygen ventilating column 40 via the liquid inlet 401 along the angleθ between the exit of the transport pipe 30 and the liquid inlet 401 of the oxygen ventilating column 40. When the nutrient solution strikes into the oxygen ventilating column 40 via the liquid inlet 401, the nutrient solution hits the oxygen ventilating column 40 of the oxygen jet device 4 to form a spinning splash by which the oxygen is ventilated through the upper ventilator 402. The nutrient solution drops to be collected to the neck 404 and then goes to hit an extension 421 of an exhaust 42 to form another splash so that oxygen is exhausted through the side ventilator 405. Finally, the nutrient solution drops to be collected inside a liquid collector 41 so that oxygen can be ventilated out from a top of the exhaust 42. Thereby, most of oxygen in the nutrient solution is ventilated out from the nutrient solution for further photosynthesis of algae microbe. Since the nutrient solution has become an oxygen-saturated liquid when passes the oxygen ventilating column 40, it is hard to conduct photosynthesis any more. Therefore, the oxygen ventilating column 40 can be made of opaque material such as stainless steel. The most of oxygen in the nutrient solution has been ventilated out when the nutrient solution is collected in the liquid collector 41. Therefore, the liquid collector 41 can be made of transparent material such as glass for further photosynthesis, or made of opaque material.

It is noted that, as shown in FIG.4, the exit of the transport pipe 30 is connected to the liquid inlet 401 along the angleθas mentioned above. The transport pipe 30 is designed according to the requirement for different algae. When the nutrient solution enters the oxygen ventilating column 40, the splash formed by jetting forms a shear force. Unduly large shear force adversely affects the cultivation of the algae or result in the death of the algae. The exit of the transport pipe 30 can be connected to the liquid inlet 401 along a particular angle so as to adjust the shear force formed when the nutrient solution is injected into the oxygen ventilating column 40. The angle can vary based on the requirements for algae. For example, the angle θ1 can be 90 degrees as shown in FIG. 4A, the angle θ2 can be 45 degrees as shown in FIG. 4B, the angle θ3 can be 0 degree as shown in FIG. 4C.

Specifically, when the algae are Spirulina, the exit of the transport pipe 30 is connected to liquid inlet 401 along the direction parallel to the tangent line T. When the algae are Haematococcus Pluvialis Flotow, which has flagellum, unduly large shear force will cut off the flagellum and adversely affect the growth of the algae or make the growing algae die. In this case, the exit of the transport pipe 30 can be adjusted with the angle less than 90n degrees, i.e., the exit of the transport pipe 30 being not parallel to the tangent line T. Thereby, when the nutrient solution enters into the oxygen ventilating column 40, the shear force is reduced to prevent the flagellum of Haematococcus Pluvialis Flotow from being broken.

Afterward, when the nutrient solution flows to the connecting piping 5 via the connector 6, the switch valve 60 may temporarily opened to clean up heavier deposits, sample the nutrient solution for testing, or act as a harvest opening. The higher liquid level of the nutrient solution collected by the liquid collecting column 41, as compared with an upper layer in the transparent piping of the photosynthesis reaction unit 1, creates a potential drop to generate the pressure so that the algae nutrient solution automatically flows into the transparent piping via the connecting pipe 5, and then the nutrient solution flows into the pressured liquid transport unit 3. Thereby, the nutrient solution can be circulated within the for algae microbe photosynthesis according to the invention to cultivate the algae microbe. After the numbers of the algae microbes in the nutrient solution reach a collectable level, the collecting valve 2 is opened to collect or the switch valve 60 is opened to collect.

During the cultivation, the nutrient solution flows into the connecting piping 5 via the connector 6. Since the connecting piping 5 has the extension pipe 52, the flowing speed of the nutrient solution slows down in the extension pipe 52. By this way, the nutrient solution stays in the connecting piping 5 for a period of time which is long enough for physiological accommodation of the algae. Any damage which might be caused by the pressured liquid transport unit 3 and the oxygen jet device 4 can therefore be eliminated, which contributes to optimal physiological status for the algae and therefore algae of increased quality.

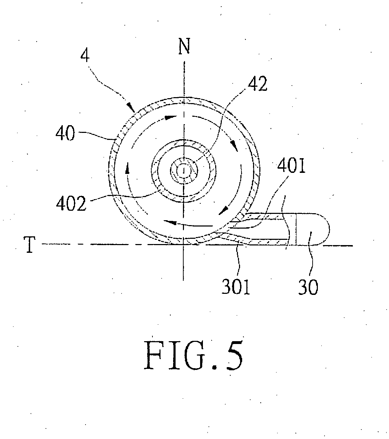

Referring to FIG. 5, in this embodiment, the exit of the liquid transport pipe 30 further has gradually reduced diameter to form a shrinking mouth 301. When the nutrient solution flows to the shrinking mouth 301 from the pressured liquid transport unit 3 under pressure, the nutrient solution is jetted higher to get into the oxygen ventilating column 40. When the nutrient solution hits the pipe wall 403, most oxygen can be ventilated without waiting for the spinning splash to drop. Therefore, the performance of ventilating oxygen improves. However, please understand this embodiment is not suitable to culture the Haematococcus Pluvialis Flotow due to possible breakage of flagellum.

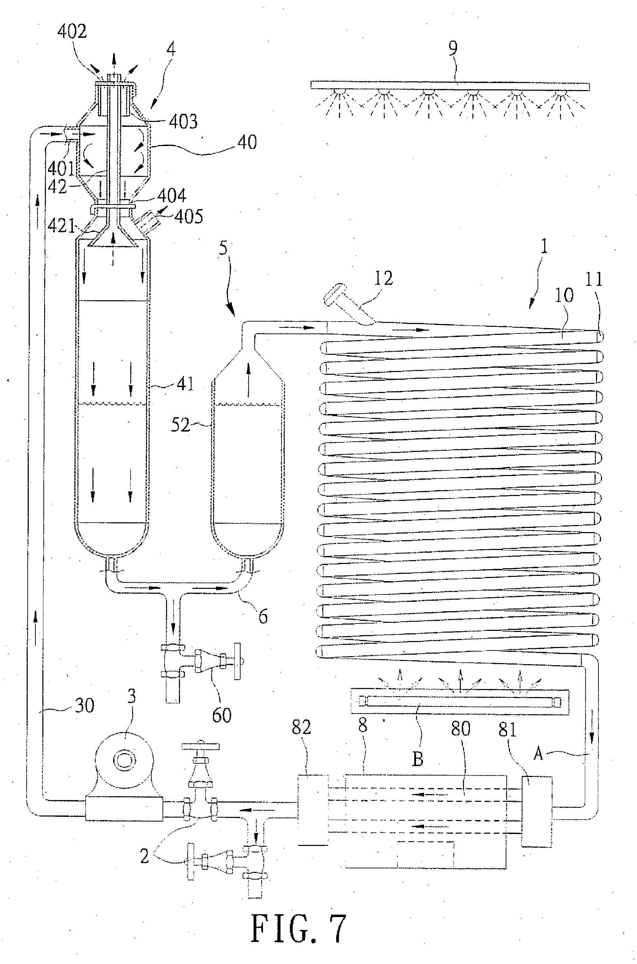

Furthermore, a temperature controlling unit 8, a water sprayer 9 and at least one light supplement unit B can be further comprised in the system of the invention, as shown in FIG. 7.

The temperature controlling unit 8 is provided with a plurality of temperature increasing and decreasing tubes 80, an inlet adapter 81 and an exit adapter 82. The temperature increasing and decreasing tubes are respectively connected to an exit of the communicating unit A and an inlet of the pressured liquid transport unit 3. The temperature controlling unit 8 heats the water within the temperature controlling unit 8 either by manually operating or automatic control. The thermal energy of water is transferred to the temperature increasing and decreasing tubes 80 for controlling the temperature of the nutrient solution. Alternatively, cold water is poured into the temperature controlling unit 8 to reduce the temperature of the nutrient solution. The location of the temperature controlling unit 8 is not limited between the collecting valve 2 and the communicating unit A. The communicating unit 8 can be located at other places as long as the purpose of controlling temperature is achieved.

The water sprayer 9 is located upon the photosynthesis reaction unit 1, and is operated either by manually operating or automatic control to reduce the temperature of the nutrient solution within the transparent piping.

As shown in FIG. 7, the light supplement unit B can be a fluorescent lamp or LED, for example. The number of the light supplement unit B may vary as needed. The light supplement unit B can be located on any side, such as upper side or lower side, of the transparent piping. In this embodiment the light supplement unit B is located on the lower side of the transparent piping, the light source of the light supplement unit B can be white light, red light or blue light. By supplying the transparent piping with proper intensity of light, the environment to culture different algae can be adjusted for the purpose of increasing the yield and lowering the production cost.

As shown in FIG. 6, in this embodiment, the connecting pipe 5 of the algae microbe photosynthesis reaction system has a straight pipe 53. One end of the connecting pipe 5 is connected to the photosynthesis reaction unit I, and the other end of the connecting pipe 5 is connected to the liquid collector 41. The algae microbe flows into the photosynthesis reaction unit 1 via the straight pipe 53 of the connecting pipe 5 to circulate for culture.

One of purposes the connecting pipe 5 is to adjust the physiological accommodation of the algae microbe in the connecting pipe 5. For the algae which need shorter time for physiological accommodation, the connecting pipe 5 with the straight pipe 53 allows the algae microbe to quickly flow into the transparent piping for circulation. For the algae which need longer time for physiological accommodation, the connecting pipe 5 with the extension tube 52 is needed to slow down the flow of the algae microbe in the connecting pipe 5 so that the algae microbe has enough time to accommodate their physiology.

Referring to FIG. 7, in this embodiment, massive oxygen generation problem can be improved. When the amount of oxygen generated increases, the amount of oxygen needed to be ventilated increases accordingly. In this embodiment, the pipe wall 403 reaches the location above the liquid inlet 401, i.e., corresponds to the liquid inlet 401. When the nutrient solution is injected into the oxygen ventilating column 40 via the liquid inlet 401 of the oxygen jet device 4 and hits the exhaust 42, part of oxygen is ventilated without waiting the spinning splash to drop, thereby increasing the amount of the oxygen ventilated. However, a few of the nutrient solution might sputters out of the upper ventilator 402 due to the splashing of the nutrient solution against the upper ventilator 402.

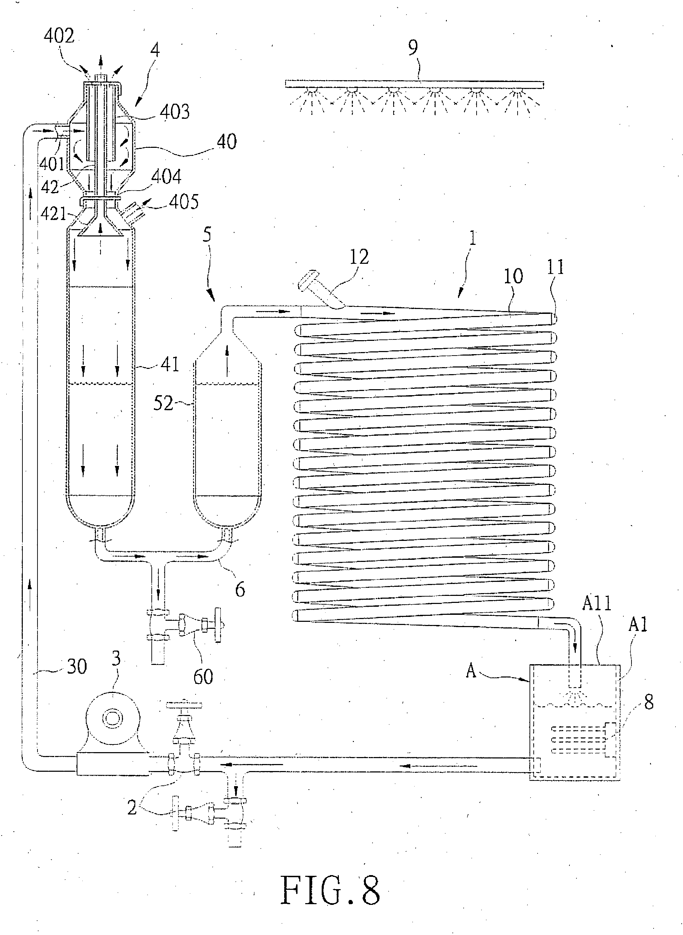

Referring to FIG. 8, in this embodiment, the location of the temperature controlling unit 8 may vary. When the communicating unit A is as also the liquid collecting sink A1, the temperature controlling unit 8 may be located inside the liquid collecting sink A1 for controlling the temperature of the nutrient solution, or the temperature controlling unit may also be located in other places more appropriate than the liquid collecting sink A1. In other words, the temperature controlling unit 8 can be arranged between the exit of the transparent piping and the entrance of the pressured liquid transport unit 3 to achieve the purpose of controlling the temperature of the nutrient solution.

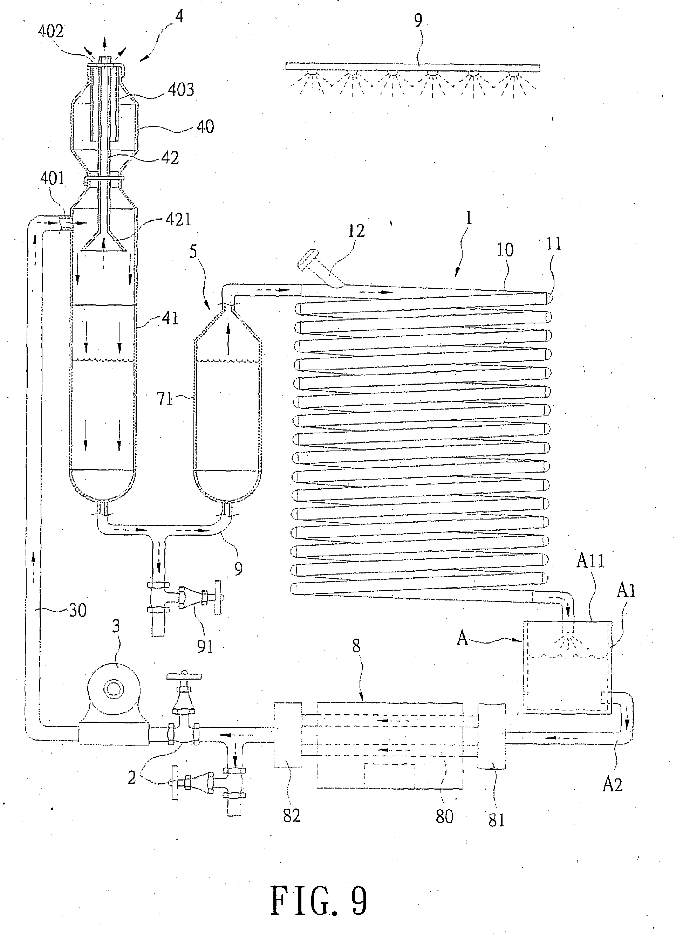

Referring to FIG. 9, for some particular algae, such as Haematococcus Pluvialis Flotow, in order to prevent large shear force from being formed when the nutrient solution is injected into the oxygen ventilating column 40 and when the spinning splash is formed, which may result in algae death. In this embodiment, the liquid inlet 401 is located at a lower section of the oxygen ventilating column 40 and above the extension 421.

When the liquid inlet 401 is at a lower height, the time to generate spinning splash from the nutrient solution is reduced so that the nutrient solution directly drops to the extension 421 and then into the liquid collector 41. By means of arranging the liquid inlet 401 at the lower section of the oxygen ventilating column 401 and connecting the exit of the transport pipe 30 to the liquid inlet 401 along the angleθ between the tangent line T and the normal line N, this would decrease damage by preventing unduly large shear force which would have adversely affects the algae growth or even leads to the algae death.

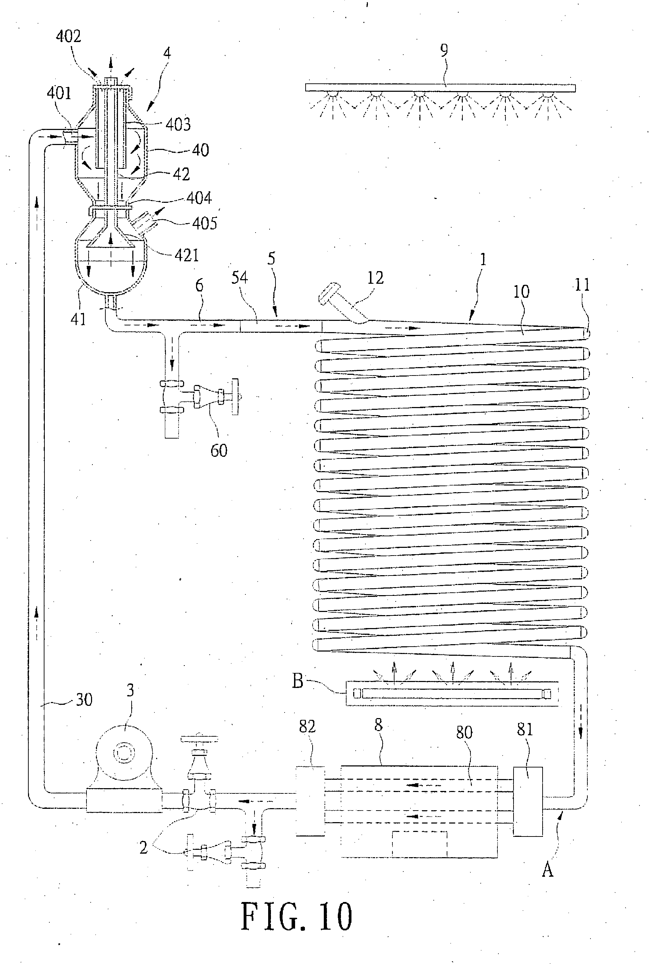

The embodiment as shown in FIG. 10 illustrates the speed-up culture for specific algae which need not no physiological accommodation. The liquid collector 41 of the oxygen jet device 4 faces the oxygen ventilating column 40 and has its lower section shortened upward. The connecting pipe 5 is a horizontal tube 54 connecting the liquid collector 41 to the transparent piping so that the nutrient solution need not have any physiological accommodation allowance for the algae and the solution would quickly flow into the transparent piping.

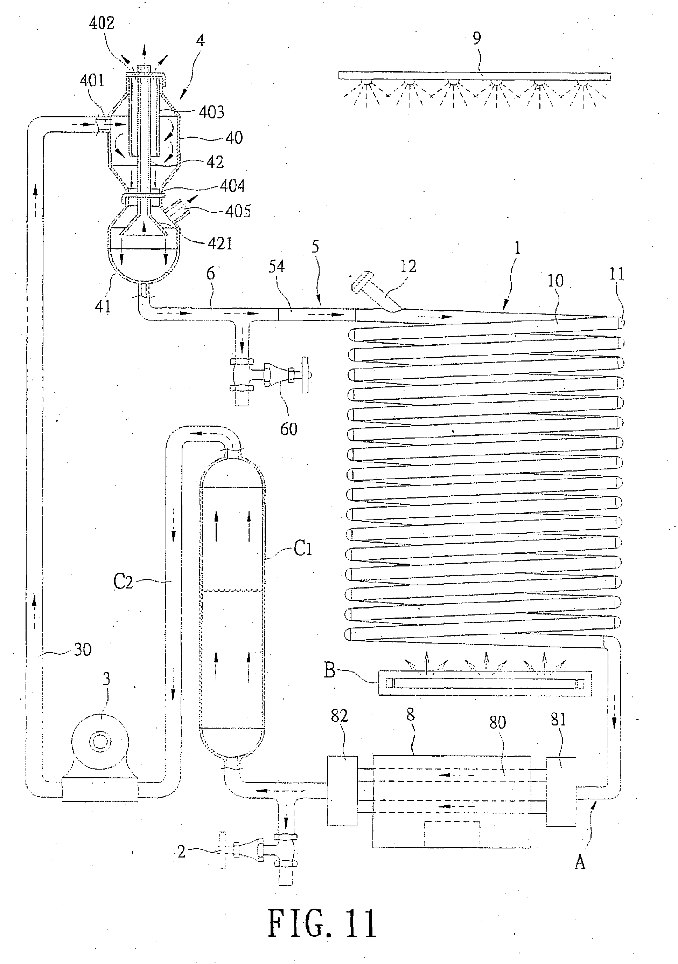

Referring to FIG. 11 and FIG. 2, the nutrient solution flowing from the oxygen ventilating column 40 down to the liquid collector 41, wherein some particular algae may stick onto walls at the lower section of the liquid collector 41 as shown in FIG. 4, because the flow of the nutrient solution slows down when passing through this location and then forced into the photosynthesis reaction unit 1 through the connecting pipe 5. The slow flow speed and the pressure result in the sticking of these particular algae onto the walls. This is like rivers have silt deposit when flowing slowly. As shown in FIG. 11, the system of the invention further comprises an adjustment column C1 and a joint C2. A bottom of the adjustment column C1 is connected to the exit of the collecting valve 2. The joint C 2 connects a top of the adjustment column C1 to the entrance of the pressured liquid transport unit 3.

When the nutrient solution flows into the adjustment column C1 and the liquid level is lifted to the highest level, the pressured liquid transport unit 3 may open to force the nutrient solution into the oxygen jet device 4 by its suction action. The liquid collector 41 has a smaller diameter at its upper part so that the nutrient solution flows into the adjustment column C1 at slower speed. However, the suction of the pressured liquid transport unit 3 reduces the possibility of deposit onto the walls and accelerates the flow of the nutrient solution into the photosynthesis reaction unit 1 after the liquid collector 41. Thereby, the circulation can be speed up and thus the yield increases. Is this case, some elements such as the adjustment column C1 and the joint C2 etc and extra space are needed. However, the increase in production cost can be covered by increased yield which is obtained by reducing the sticking of particular algae on the walls. Therefore, the addition of the extra elements and space would not be a disadvantage in terms of total profit, considering its benefit.

According to another preferred embodiment, referring to FIG. 2, FIG. 4A, FIG. 4B and FIG. 4C, this invention also provides a system for photosynthesis reaction of algae microbe, comprising: a photosynthesis reaction unit 1, which is a transparent piping; a communicating unit A, connected to an exit of the transparent piping; a collecting valve 2, connected to an exit of the communicating unit A; a pressured liquid transport unit 3, an entrance of which is connected to the collecting valve 2, and the pressured liquid transport unit 3 has a transport pipe 30; an oxygen jet device 4, being a hollow pipe and comprising the oxygen ventilating column 40 and a liquid collector 41 at a lower end thereof, the oxygen ventilating column 40 having a liquid inlet 401, an upper ventilator 402, and a pipe wall 403, the exit of the transport pipe 30 communicating with the liquid inlet 401, wherein the exit of the transport pipe 30 being connected to the liquid inlet 401 along an included angle defined on a horizontal cross-sectional surface of the oxygen jet device 4 between an axial line of the transport pipe 30 and a normal line N passing through a central point of the oxygen jet device 4.

Preferably, the included angle above is adjustable according to different kinds of algae. The upper ventilator 402 can be located at a top of the oxygen ventilating column 40, and the pipe wall 403 extends downward from the upper ventilator 402. Moreover, the system also includes a connecting pipe 5, which is a close pipe. The connecting pipe 5 can be used to connect the liquid collector 41 to the photosynthesis reaction unit 1.

In light of the above, the system and method for algae microbe according to the invention offer the following advantages.

First, an enclosed multi-piping configuration formed by the photosynthesis reaction unit 1, the communicating unit A, the collecting valve 2, the pressured liquid transport unit 3, the transport pipe 30, the oxygen jet device 4 and the connecting pipe 5 allow the enclosed nutrient solution to circulate therein for photosynthesis and oxygen ventilation. Its unique features such as small use of area, reduced energy requirement and no restrain from everyday weather greatly increases the algae yield and quality because the enclosed algae can be protected from pollutions especially when in the liquid collecting sink A1 of the communicating unit A. Additionally, the system of the invention can also be used in a partially enclosed environment.

Second, the entrance of the transport pipe 30 is connected to the liquid inlet 401 along the angle θ between the normal line N and the tangent line T of the oxygen jet device 4. The angle θ may vary as needed for different algae in order to prevent any unduly large shear force formed by spinning splash due to the injecting of the nutrient solution.

Third, the arrangement of the liquid inlet 401, the upper ventilator 402 and the pipe wall 403, also the arrangement of the exhaust 42 and the extension 421 make the oxygen formed in the nutrient solution easy to ventilate, thereby increasing the yield and is favorable for mass production.

Fourth, the assembly of the oxygen ventilating column 40 and the liquid collector 41 to form the oxygen jet device 4 is easy and strong, and therefore has lower cost in terms of assembling and maintenance.

Fifth, the assembly of the oxygen column 40 and the liquid collector 41, and the design of the transparent piping are easy for cleaning and maintenance so as to ensure the effect of photosynthesis and quality of algae.

Sixth, the arrangement of the temperature controlling unit 8, the water sprayer 9 and the light supplement unit B allows proper temperature adjustment and light intensity for the nutrient solution according to geography, seasons and weathers.

It should be apparent to those skilled in the art that the above description is only illustrative of specific embodiments and examples of the invention. The invention should therefore cover various modifications and variations made to the herein-described structure and operations of the invention, provided they fall within the scope of the invention as defined in the following appended claims.

## Claims

1. A method for photosynthesis reaction of algae microbe, comprising:
(a) providing a photosynthesis reaction unit (1), comprising a transparent piping, a communicating unit (A), a collecting valve (2), a pressured liquid transport unit (3) with a transport pipe (30), an oxygen jet device (4) with a liquid inlet (40), and a connecting pipe (5);
(b) injecting an algae and microbe nutrient solution into the transparent piping, circulating the nutrient solution within the transparent piping for photosynthesis to generate oxygen, wherein the nutrient solution flows to the communicating unit (A), the collecting valve (2), and the pressured liquid transport unit (3);
(c) determining an included angle on a horizontal cross-sectional surface of the oxygen jet device (4) between an axial line of the transport pipe (30) and a normal line (N) passing through a central point of the oxygen jet device (4), wherein the included angle is adjustable according to different kinds of algae;
(d) opening the pressured liquid transport unit (3) to force the nutrient solution to flow toward the oxygen jet device (4) from the transport pipe (30), wherein the nutrient solution flows into the oxygen jet device (4) from the transport pipe (30) via the liquid inlet (401) along the included angle and caused a shear force on the algae, and is splashed into the oxygen jet device (4) to form a spinning splash by which the oxygen is ventilated, wherein the shear force is reduced when the included angle is smaller;
(e) collecting the nutrient solution in the oxygen jet device (4) and the connecting pipe (5);
(f) flowing the nutrient solution into the transparent piping for further photosynthesis; and
(g) taking the nutrient solution from the collecting valve (2).

2. The method of claim 1, wherein at step (b) the nutrient solution flows up and down to circulate within the transparent piping.

3. The method of claim 1, wherein at step (b) further providing a temperature controlling unit (8), wherein the nutrient solution flows to the pressured liquid transport unit (3) after the temperature controlling unit (8); at steps (b) and (g) further providing a water sprayer (9), wherein the water sprayer (9) sprays over the transparent piping; and at steps (b) and (g) further providing at least one light supplement unit (B) to radiate the transparent piping.

4. The method of claim 1, wherein at step (g) the communicating unit (A) further has a liquid collecting sink (A1), the nutrient solution being taken from the liquid collecting sink (A1) after flowing into the liquid collecting sink (A1).

5. A system for photosynthesis reaction of algae microbe, comprising:
a photosynthesis reaction unit (1), which is a transparent piping;
a communicating unit (A), connected to an exit of the transparent piping;
a collecting valve (2), connected to an exit of the communicating unit (A);
a pressured liquid transport unit (3), an entrance of which is connected to the collecting valve (2), and the pressured liquid transport unit (3) has a transport pipe (30);
an oxygen jet device (4), being a hollow pipe and comprising the oxygen ventilating column (40) and a liquid collector (41) at a lower end thereof, the oxygen ventilating column (40) having a liquid inlet (401), an upper ventilator (402), and a pipe wall (403), the exit of the transport pipe (30) communicating with the liquid inlet (401), wherein the exit of the transport pipe (30) being connected to the liquid inlet (401) along an included angle defined on a horizontal cross-sectional surface of the oxygen jet device (4) between an axial line of the transport pipe (30) and a normal line (N) passing through a central point of the oxygen jet device (4), wherein the included angle is adjustable according to different kinds of algae, the upper ventilator (402) being located at a top of the oxygen ventilating column (40), and the pipe wall (403) extending downward from the upper ventilator (402); and
a connecting pipe (5), being a close pipe and connected the liquid collector (41) to the photosynthesis reaction unit (1).

6. The system of claim 5, wherein the photosynthesis reaction unit (1) comprises a plurality of straight pipes (10) and a plurality of bent pipes (11), the straight pipes (10) and the bent pipes (11) being arranged in series in turns to form a dual-row tilt spiral transparent piping, and an auxiliary opening (12) being formed at the upper most part of the transparent piping.

7. The system of claim 5, wherein the communicating unit (A) is a connecting pipe (5) connecting the transparent piping to the collecting valve (2), or the communicating unit (A) has a liquid flowing pipe (A2) and a liquid collecting sink (A1) with an upward opening, the opening corresponding to the exit of the transparent piping, and the liquid flowing pipe (A2) being connected the liquid collecting sink (A1) to the entrance of the collecting valve (2).

8. The system of claim 5, wherein the exit of the transport pipe (30) further has gradually reduced diameter to form a shrinking mouth (301).

9. The system of claim 5, wherein the oxygen jet device (4) further comprises an exhaust (42) assembled inside the oxygen ventilating column (40), an upper end of the exhaust (42) penetrating through the pipe wall (403), a lower end of the exhaust (42) having the extension opposite to the interior of the side ventilator (405) (405).

10. The system of claim 5, wherein the connecting pipe (5) is an extending pipe (52), a straight pipe (53) or a horizontal pipe which connects the liquid collector (41) to the transparent piping.

11. The system of claim 5, further comprising a connector (6) connecting a bottom of the liquid collector (41) to a bottom of the connecting pipe (5), and the connector (6) further includes a switch valve (60).

12. The system of claim 5, further comprising a temperature controlling unit (8), a water sprayer (9) and at least one light supplement unit (B), wherein the temperature controlling unit (8) connects the exit of the transparent piping to the entrance of the collecting valve (2), the water sprayer (9) being located above the photosynthesis reaction unit (1) and the light supplement unit (B) being located on one side of the transparent piping.

13. The system of claim 5, further comprises an adjustment column (C1) and a joint (C2), a bottom of the adjustment column (C1) being connected to the exit of the collecting valve (2), the joint (C2) connecting a top of the adjustment column (C1) to the entrance of the pressured liquid transport unit (3).

## Patentansprüche

1. Ein Verfahren zur Photosynthese-Reaktion einer Algen-Mikrobe, aufweisend:
a) Bereitstellen einer Photosynthese-Reaktionseinheit (1), aufweisend eine transparente Leitung, eine Verbindungseinheit (A), ein Sammelventil (2), eine Druck-Flüssigkeitstransporteinheit (3) mit einer Transportleitung (30), eine Sauerstoffausströmvorrichtung (4) mit einem Flüssigkeitseinlass (40) und eine Verbindungsleitung (5),
b) Einspeisen einer Algen-und-Mikrobe-Nährlösung in die transparente Leitung, Zirkulieren der Nährlösung innerhalb der transparenten Leitung zur Photosynthese, um Sauerstoff zu erzeugen, wobei die Nährlösung zu der Verbindungseinheit (A), dem Sammelventil (2) und der Druck-Flüssigkeitstransporteinheit (3) strömt,
c) Ermitteln eines eingeschlossenen Winkels in einer horizontalen Querschnittsfläche der Sauerstoffausströmvorrichtung (4) zwischen einer axialen Linie der Transportleitung (30) und einer normalen Linie (N), welche einen zentralen Punkt der Sauerstoffausströmvorrichtung (4) durchläuft, wobei der eingeschlossene Winkel gemäß verschiedenen Arten von Algen einstellbar ist,
d) Öffnen der Druck-Flüssigkeitstransporteinheit (3), um die Nährlösung zu zwingen von der Transportleitung (30) zu der Sauerstoffausströmvorrichtung (4) zu strömen, wobei die Nährlösung von der Transportleitung (30) über den Flüssigkeitseinlass (401) entlang des eingeschlossenen Winkels in die Sauerstoffausströmvorrichtung (4) strömt, wobei eine Scherkraft auf die Algen verursacht wird, und in die Sauerstoffausströmvorrichtung (4) gespritzt wird, um ein wirbelndes Spritzen auszubilden, durch welches der Sauerstoff entlüftet wird, wobei die Scherkraft reduziert ist, wenn der eingeschlossene Winkel kleiner ist,
e) Sammeln der Nährlösung in der Sauerstoffausströmvorrichtung (4) und der Verbindungsleitung (5),
f) Strömen der Nährlösung in die transparente Leitung zur weiteren Photosynthese, und
g) Entnehmen der Nährlösung aus dem Sammelventil (2).

2. Das Verfahren nach Anspruch 1, wobei in Schritt (b) die Nährlösung nach oben und unten strömt, um innerhalb der transparenten Leitung zu zirkulieren.

3. Das Verfahren nach Anspruch 1, wobei in Schritt (b) ferner eine Temperatursteuereinheit (8) bereitgestellt ist, wobei die Nährlösung nach der Temperatursteuereinheit (8) zu der Druck-Flüssigkeitstransporteinheit (3) strömt, wobei in Schritt (b) und (g) ferner ein Wasser-Sprüher (9) bereitgestellt ist, wobei der Wasser-Sprüher (9) über die transparente Leitung sprüht, und wobei in Schritt (b) und (g) ferner zumindest eine Lichtergänzungseinheit (B) bereitgestellt ist, um die transparente Leitung zu bestrahlen.

4. Das Verfahren nach Anspruch 1, wobei in Schritt (g) die Verbindungseinheit (A) ferner ein Flüssigkeitssammelbecken (A1) hat, wobei die Nährlösung aus dem Flüssigkeitssammelbecken (A1) entnommen wird, nachdem sie in das Flüssigkeitssammelbecken (A1) geströmt ist.

5. Ein System zur Photosynthese-Reaktion einer Algen-Mikrobe, aufweisend:
eine Photosynthese-Reaktionseinheit (1), welche eine transparente Leitung ist,
eine Verbindungseinheit (A), welche mit einem Ausgang der transparenten Leitung verbunden ist,
ein Sammelventil (2), welches mit einem Ausgang der Verbindungseinheit (A) verbunden ist,
eine Druck-Flüssigkeitstransporteinheit (3), von welcher ein Eingang mit dem Sammelventil (2) verbunden ist, und wobei die Druck-Flüssigkeitstransporteinheit (3) eine Transportleitung (30) hat,
eine Sauerstoffausströmvorrichtung (4), welche eine hohle Leitung ist und die Sauerstoffentlüftungssäule (40) und einen Flüssigkeitssammler (41) an einem unteren Ende davon aufweist, wobei die Sauerstoffentlüftungssäule (40) einen Flüssigkeitseinlass (401), einen oberen Entlüfter (402) und eine Leitungswand (403) hat, wobei der Ausgang der Transportleitung (30) mit dem Flüssigkeitseinlass (401) in Verbindung ist, wobei der Ausgang der Transportleitung (30) mit dem Flüssigkeitseinlass (401) entlang eines eingeschlossenen Winkels verbunden ist, welcher in einer horizontalen Querschnittsfläche der Sauerstoffausströmvorrichtung (4) zwischen einer axialen Linie der Transportleitung (30) und einer normalen Linie (N), welche einen zentralen Punkt der Sauerstoffausströmvorrichtung (4) durchläuft, definiert ist, wobei der eingeschlossene Winkel gemäß verschiedenen Arten von Algen einstellbar ist, wobei der obere Entlüfter (402) an einem Oberteil der Sauerstoffentlüftungssäule (40) angeordnet ist und wobei sich die Leitungswand (403) von dem oberen Entlüfter (402) nach unten erstreckt, und
eine Verbindungsleitung (5), welche eine geschlossene Leitung ist und den Flüssigkeitssammler (41) mit der Photosynthese-Reaktionseinheit (1) verbindet.

6. Das System nach Anspruch 5, wobei die Photosynthese-Reaktionseinheit (1) eine Mehrzahl von geraden Leitungen (10) und eine Mehrzahl von gebogenen Leitungen (11) aufweist, wobei die geraden Leitungen (10) und die gebogenen Leitungen (11) in Reihe im Wechsel angeordnet sind, um eine zweireihige geneigte spiralförmige transparente Leitung auszubilden, und wobei eine Hilfsöffnung (12) an dem obersten Teil der transparenten Leitung ausgebildet ist.

7. Das System nach Anspruch 5, wobei die Verbindungseinheit (A) eine Verbindungsleitung (5) ist, welche die transparente Leitung mit dem Sammelventil (2) verbindet, oder wobei die Verbindungseinheit (A) eine Flüssigkeitsströmungsleitung (A2) und ein Flüssigkeitssammelbecken (A1) mit einer Öffnung nach oben hat, wobei die Öffnung dem Ausgang der transparenten Leitung entspricht und wobei die Flüssigkeitsströmungsleitung (A2) das Flüssigkeitssammelbecken (A1) mit dem Eingang des Sammelventils (2) verbindet.

8. Das System nach Anspruch 5, wobei der Ausgang der Transportleitung (30) ferner einen allmählich reduzierten Durchmesser hat, um eine kleiner werdende Mündung (301) auszubilden.

9. Das System nach Anspruch 5, wobei die Sauerstoffausströmvorrichtung (4) ferner einen Auslass (42) aufweist, welcher innerhalb der Sauerstoffentlüftungssäule (40) montiert ist, wobei ein oberes Ende des Auslasses (42) die Leitungswand (403) durchdringt, wobei ein unteres Ende des Auslasses (42) die Ausweitung gegenüber des Innenteils des Seitenentlüfters (405) hat.

10. Das System nach Anspruch 5, wobei die Verbindungsleitung (5) eine verbreiterte Leitung (52), eine gerade Leitung (53) oder eine horizontale Leitung ist, welche den Flüssigkeitssammler (41) mit der transparenten Leitung verbindet.

11. Das System nach Anspruch 5, ferner aufweisend einen Verbinder (6), welcher ein Unterteil des Flüssigkeitssammlers (41) mit einem Unterteil der Verbindungsleitung (5) verbindet, und wobei der Verbinder (6) ferner ein Umschaltventil (60) aufweist.

12. Das System nach Anspruch 5, ferner aufweisend eine Temperatursteuereinheit (8), einen Wasser-Sprüher (9) und zumindest eine Lichtergänzungseinheit (B), wobei die Temperatursteuereinheit (8) den Ausgang der transparenten Leitung mit dem Eingang des Sammelventils (2) verbindet, wobei der Wasser-Sprüher (9) über der Photosynthese-Reaktionseinheit (1) angeordnet ist und wobei die Lichtergänzungseinheit (B) auf einer Seite der transparenten Leitung angeordnet ist.

13. Das System nach Anspruch 5, ferner aufweisend eine Einstellsäule (C1) und eine Verbindung (C2), wobei ein Unterteil der Einstellsäule (C1) mit dem Ausgang des Sammelventils (2) verbunden ist, wobei die Verbindung (C2) ein Oberteil der Einstellsäule (C1) mit dem Eingang der Druck-Flüssigkeitstransporteinheit (3) verbindet.

## Revendications

1. Procédé pour une réaction de photosynthèse de microbe de type algue, comprenant :
(a) la fourniture d'une unité de réaction de photosynthèse (1), comprenant une tuyauterie transparente, une unité de communication (A), une vanne de collecte (2), une unité de transport de liquide sous pression (3) avec un tuyau de transport (30), un dispositif de jet d'oxygène (4) avec une entrée de liquide (40), et un tuyau de liaison (5) ;
(b) l'injection d'une substance nutritive pour algue et microbe dans la tuyauterie transparente, la circulation de la solution de substance nutritive dans la tuyauterie transparente pour une photosynthèse afin de générer de l'oxygène, dans lequel la solution de substance nutritive s'écoule vers l'unité de communication (A), la vanne de collecte (2), et l'unité de transport de liquide sous pression (3) ;
(c) la détermination d'un angle inclus sur une surface en coupe horizontale du dispositif de jet d'oxygène (4) entre une ligne axiale du tuyau de transport (30) et une ligne normale (N) passant par un point central du dispositif de jet d'oxygène (4), dans lequel l'angle inclus est ajustable en fonction des différents types d'algues ;
(d) l'ouverture de l'unité de transport de liquide sous pression (3) pour forcer la solution de substance nutritive à s'écouler vers le dispositif de jet d'oxygène (4) à partir du tuyau de transport (30), dans lequel la solution de substance nutritive s'écoule dans le dispositif de jet d'oxygène (4) à partir du tuyau de transport (30) à travers l'entrée de liquide (401) le long de l'angle inclus et provoque une force de cisaillement sur l'algue, et est projeté dans le dispositif de jet d'oxygène (4) pour former une projection rotative par laquelle l'oxygène est ventilé, dans lequel la force de cisaillement est réduite lorsque l'angle inclus est plus petit ;
(e) la collecte de la solution de substance nutritive dans le dispositif de jet d'oxygène (4) et le tuyau de liaison (5) ;
(f) l'écoulement de la solution de substance nutritive dans la tuyauterie transparente pour une photosynthèse supplémentaire ; et
(g) le prélèvement de la solution de substance nutritive à partir de la vanne de collecte (2).

2. Procédé selon la revendication 1, dans lequel, à l'étape (b) la solution de substance nutritive s'écoule vers le haut et vers le bas pour circuler dans la tuyauterie transparente.

3. Procédé selon la revendication 1, dans lequel, à l'étape (b), une unité de régulation de température (8) est prévue en plus, dans lequel la solution de substance nutritive s'écoule vers l'unité de transport de liquide sous pression (3) après l'unité de régulation de température (8) ; aux étapes (b) et (g), un pulvérisateur d'eau (9) est prévu en plus, dans lequel le pulvérisateur d'eau (9) effectue des pulvérisations sur la tuyauterie transparente ; et aux étapes (b) et (g), au moins une lampe (B) est prévue en plus pour appliquer un rayonnement à la tuyauterie transparente.

4. Procédé selon la revendication 1, dans lequel, à l'étape (g), l'unité de communication (A) comporte en outre une cuve de collecte de liquide (A1), la solution de substance nutritive étant prélevée de la cuve de collecte de liquide (A1) après son écoulement dans la cuve de collecte de liquide (A1).

5. Système pour une réaction de photosynthèse de microbe de type algue, comprenant :
une unité de réaction de photosynthèse (1), qui est une tuyauterie transparente ;
une unité de communication (A), reliée à une sortie de la tuyauterie transparente ;
une vanne de collecte (2), reliée à une sortie de l'unité de communication (A) ;
une unité de transport de liquide sous pression (3), dont une entrée est reliée à la vanne de collecte (2), et l'unité de transport de liquide sous pression (3) comporte un tuyau de transport (30) ;
un dispositif de jet d'oxygène (4), qui est un tuyau creux et qui comprend la colonne de ventilation d'oxygène (40) et un collecteur de liquide (41) à une extrémité inférieure de celui-ci, la colonne de ventilation d'oxygène (40) comportant une entrée de liquide (401), un ventilateur supérieur (402), et une paroi de tuyau (403), la sortie du tuyau de transport (30) communiquant avec l'entrée de liquide (401), dans lequel la sortie du tuyau de transport (30) est reliée à l'entrée de liquide (401) selon un angle inclus défini sur une surface en coupe horizontale du dispositif de jet d'oxygène (4) entre une ligne axiale du tuyau de transport (30) et une ligne normale (N) passant par un point central du dispositif de jet d'oxygène (4), dans lequel l'angle inclus est ajustable en fonction des différents types d'algue, le ventilateur supérieur (402) étant situé en haut de la colonne de ventilation d'oxygène (40), et la paroi de tuyau (403) s'étendant vers le bas à partir du ventilateur supérieur (402) ; et
un tuyau de liaison (5), qui est un tuyau fermé et reliant le collecteur de liquide (41) à l'unité de réaction de photosynthèse (1).

6. Système selon la revendication 5, dans lequel l'unité de réaction de photosynthèse (1) comprend une pluralité de tuyaux droits (10) et une pluralité de tuyaux cintrés (11), les tuyaux droits (10) et les tuyaux cintrés (11) étant agencés en série tour à tour pour former une tuyauterie transparente en spirale inclinée à double rangée, et une ouverture auxiliaire (12) étant formée au niveau de la partie la plus haute de la tuyauterie transparente.

7. Système selon la revendication 5, dans lequel l'unité de communication (A) est un tuyau de liaison (5) reliant la tuyauterie transparente à la vanne de collecte (2), ou l'unité de communication (A) comporte un tuyau d'écoulement de liquide (A2) et une cuvette de collecte de liquide (A1) avec une ouverture vers le haut, l'ouverture correspondant à la sortie de la tuyauterie transparente, et le tuyau d'écoulement de liquide (A2) reliant la cuve de collecte de liquide (A1) à l'entrée de la vanne de collecte (2).

8. Système selon la revendication 5, dans lequel la sortie du tuyau de transport (30) a en outre un diamètre qui diminue graduellement pour former une embouchure qui rétrécit (301).

9. Système selon la revendication 5, dans lequel le dispositif de jet d'oxygène (4) comprend en outre un élément d'échappement (42) assemblé à l'intérieur de la colonne de ventilation d'oxygène (40), une extrémité supérieure de l'élément d'échappement (42) pénétrant à travers la paroi de tuyau (403), une extrémité inférieure de l'élément d'échappement (42) ayant l'extension face à l'intérieur du ventilateur latéral (405).

10. Système selon la revendication 5, dans lequel le tuyau de liaison (5) est un tuyau élargi (52), un tuyau droit (53) ou un tuyau horizontal qui relie le collecteur de liquide (41) à la tuyauterie transparente.

11. Système selon la revendication 5, comprenant en outre un raccord (6) reliant une partie inférieure du collecteur de liquide (41) à une partie inférieure du tuyau de liaison (5), et le raccord (6) comprend en outre une vanne de commutation (60).

12. Système selon la revendication 5, comprenant en outre une unité de régulation de température (8), un pulvérisateur d'eau (9) et au moins une lampe (B), dans lequel l'unité de régulation de température (8) relie la sortie de la tuyauterie transparente à l'entrée de la vanne de collecte (2), le pulvérisateur d'eau (9) étant situé au-dessus de l'unité de réaction de photosynthèse (1) et la lampe (B) étant située d'un côté de la tuyauterie transparente.

13. Système selon la revendication 5, comprenant en outre une colonne d'ajustement (C1) et un élément de jonction (C2), une partie inférieure de la colonne d'ajustement (C1) étant reliée à la sortie de la vanne de collecte (2), l'élément de jonction (C2) reliant une partie supérieure de la colonne d'ajustement (C1) à l'entrée de l'unité de transport de liquide sous pression (3).
